# EUROPEAN PATENT SPECIFICATION

(11) **EP 2 543 366 B1**
(45) Date of publication and mention of the grant of the patent: **24.10.2018**
(21) Application number: 11750752.5
(22) Date of filing: 03.03.2011
(51) Int. Cl.: A61K 9/70, A61K 31/045, A61K 47/38, A61K 9/00

(54) **FILM PREPARATION CONTAINING MEDICAMENT WITH UNPLEASANT TASTE**
FILMPRÄPARAT MIT MEDIKAMENT MIT UNANGENEHMEM GESCHMACK
PRÉPARATION DE FILM CONTENANT UN MÉDICAMENT AU GOÛT DÉSAGRÉABLE

(30) Priority: 03.03.2010 JP 2010047071
(43) Date of publication of application: 09.01.2013
(73) Proprietor: Kyukyu Pharmaceutical Co., Ltd., Tokyo 103-0023 (JP)
(72) Inventor: AWAMURA, Tsutomu, Imizu-shi Toyama 939-0351 (JP); NISHIKAWA, Hisanobu, Imizu-shi Toyama 939-0351 (JP); KOKAJI, Kazuhiko, Imizu-shi Toyama 939-0351 (JP); ISHISE, Akihiro, Imizu-shi Toyama 939-0351 (JP); ARAI, Takayuki, Fuji-shi Shizuoka 417-8650 (JP); MIURA, Seiji, Fuji-shi Shizuoka 417-8650 (JP); INAGI, Toshio, Tokyo 103-8433 (JP)
(74) Representative: Hartz, Nikolai
(86) International application number: PCT/JP2011/054914
(87) International publication number: WO 2011/108643

(56) References cited:
- EP-A1- 1 504 765
- EP-A1- 1 752 127
- WO-A1-2009/072572
- WO-A1-2010/023874
- JP-A- 2002 525 306
- JP-A- 2003 527 410
- JP-A- 2005 232 072
- JP-A- 2005 263 704
- JP-A- 2005 289 867
- JP-A- 2007 254 340
- JP-A- 2008 517 935
- JP-A- 2009 007 310
- JP-B2- 3 706 204
- US-A1- 2006 039 958
- YASUHIKO NAKAMURA: 'Masking of Bitter Taste and Sustained Release Preparation' JOURNAL OF PHARMACEUTICAL SCIENCE AND TECHNOLOGY, JAPAN 1079-7440 vol. 65, no. 3, 2005, pages 159 - 164, XP008142946

## Description

### Technical Field

The present invention relates to a film preparation in which an unpleasant taste of a medicament is masked, a method for producing the same, and a method for masking an unpleasant taste in a film preparation containing a medicament having an unpleasant taste and wherein the medicament having an unpleasant taste is loxoprofen sodium hydrate or pitavastatin calcium.

### Background Art

In recent years, various film preparations have been studied and developed as pharmaceutical dosage forms which are convenient when they are carried (Patent Document 1) . Convenience of carrying pharmaceuticals is a great advantage because it leads to easiness of taking the pharmaceuticals when outside the home, for example, and further leads to improvement of compliance with taking the pharmaceuticals. In particular, a film preparation which is dissolved in the oral cavity can be taken without water and hence can be taken even in a situation where taking with water is difficult, such as during commuting or meeting. Therefore, such a preparation is particularly useful. However, in the case of a film preparation containing a medicament having an unpleasant taste such as a bitter taste, a harsh taste, or an astringent taste, a bad sensation upon taking the preparation based on the unpleasant taste causes, for example, rejection of taking the preparation by a patient, resulting in significant deterioration of the advantage of the film preparation, i.e., good compliance with taking the preparation. Therefore, various studies have been made on a technology for masking an unpleasant taste of the film preparation containing a medicament having an unpleasant taste.

As the technology for masking an unpleasant taste in the film preparation, there have been reported, for example, a technology involving for blending a flavoring agent such as an ion-exchange resin (Patent Document 2) and a technology involving providing a water-swellable gel-forming layer as an outermost layer in a film preparation including a medicament-containing layer and a water-swellable gel-forming layer (Patent Document 3).

### Prior art documents

### Patent Documents

[Patent Document 1] JP-A-2004-43450
[Patent Document 2] JP-A-2003-527410
[Patent Document 3] WO 02/087622 A1

### Summary of Invention

### Technical Problem

However, all the conventional film preparations have insufficient masking effects.

The present invention is to provide a film preparation in which an unpleasant taste derived from a medicament is masked and wherein the medicament having an unpleasant taste is loxoprofen sodium hydrate or pitavastatin calcium.

### Solution to Problem

The inventors of the present invention have conducted various researches to achieve the foregoing object, and they have surprisingly found that a noticeable effect of masking an unpleasant taste of a medicament is exerted by a film preparation including coating layers containing no terpene formed on both sides of a medicament-containing layer containing a medicament having an unpleasant taste and a terpene. The content of the terpene in the claimed film preparation is 0.2 to 15% by mass with respect to the whole film preparation, and the medicament having an unpleasant taste is loxoprofen sodium hydrate or pitavastatin calcium. Thus, the present invention has been accomplished.

That is, the present invention provides a film preparation, including coating layers containing no terpene formed on both sides of a medicament-containing layer containing a medicament having an unpleasant taste (namely loxoprofen sodium hydrate or pitavastatin calcium) and a terpene.

The present invention also provides a method for masking an unpleasant taste in a film preparation containing a medicament having an unpleasant taste (namely loxoprofen sodium hydrate or pitavastatin calcium), the method including forming coating layers containing no terpene on both sides of a medicament-containing layer containing a said claimed medicament having an unpleasant taste and a terpene.

The present invention also provides a method for producing a film preparation in which an unpleasant taste derived from a medicament having an unpleasant taste (namely loxoprofen sodium hydrate or pitavastatin calcium) is masked, the method including the step of forming coating layers containing no terpene on both sides of a medicament-containing layer containing a medicament having an unpleasant taste and a terpene.

### Effects of Invention

The film preparation of the present invention can reduce a bad sensation of taking the preparation, because an unpleasant taste derived from a medicament is masked.

### Brief Description of Drawings

[FIG. 1] A cross-sectional view illustrating one embodiment of a film preparation of the present invention.
[FIG. 2] A cross-sectional view illustrating another embodiment of a film preparation of the present invention.
[FIG. 3] A cross-sectional view illustrating another embodiment of a film preparation of the present invention.
[FIG. 4] A cross-sectional view illustrating another embodiment of a film preparation of the present invention.
[FIG. 5] A cross-sectional view illustrating another embodiment of a film preparation of the present invention.
[FIG. 6] A cross-sectional view illustrating another embodiment of a film preparation of the present invention.
[FIG. 7] A cross-sectional view illustrating another embodiment of a film preparation of the present invention.

### Description of Embodiments

A film preparation of the present invention is a multi-layer film preparation including "coating layers" formed on both sides of a "medicament-containing layer" containing a medicament having an unpleasant taste. The "medicament-containing layer" contains the medicament having an unpleasant taste and a terpene, while the "coating layer" is characterized by containing no terpene. It should be noted that, in the present invention, the term "coating layer" means a layer containing no medicament having an unpleasant taste (that is, a layer other than the "medicament-containing layer"). Therefore, the film preparation of the present invention is a film preparation which includes coating layers formed on both sides of a medicament-containing layer containing a medicament having an unpleasant taste (namely loxoprofen sodium hydrate or pitavastatin calcium) and contains a terpene only in the medicament-containing layer.

The dose and content of the medicament having an unpleasant taste (namely loxoprofen sodium hydrate or pitavastatin calcium) contained in the film preparation of the present invention described above are preferably as follows.

The film preparation of the present invention is one which allows pitavastatin calcium to be taken in an amount of 1 to 4 mg per dose. The film preparation of the present invention contains pitavastatin calcium in an amount of preferably 1 to 4 mg, more preferably 1 mg, 2 mg, or 4 mg per film preparation.

The film preparation of the present invention is one which allows loxoprofen sodium hydrate to be taken in an amount of 60 to 120 mg per dose in terms of anhydrous loxoprofen sodium, and the daily dose is preferably divided into one to three doses. The film preparation of the present invention contains anhydrous loxoprofen sodium in an amount of preferably 10 to 60 mg, more preferably 10 mg, 20 mg, 30 mg, or 60 mg per film preparation.

In the present invention, the term "a terpene" represents a concept including a terpene and an essential oil containing a terpene. Specific examples of the terpene include limonene, pinene, camphene, cymene, cineol, citronellol, geraniol, nerol, linalool, menthol, terpineol, rhodinol, borneol, isoborneol, menthone, camphor, eugenol, cinnzeylanol and the like. These terpenes have a single stereoisomer and a mixture thereof . In the present invention, the terpen is preferably one or more selected from the group consisting of geraniol, menthol, borneol, camphor, and eugenol, more preferably one or more selected from the group consisting of menthol, camphor, borneol, and eugenol. It should be noted that a preferred menthol is dl-menthol or 1-menthol, a preferred borneol is d-borneol, and a preferred camphor is dl-camphor. Examples of the essential oil containing a terpene include orange peel oil, orange oil, mentha oil, white camphor oil, eucalyptus oil, turpentine oil, lemon oil, ginger oil, clove oil, cinnamon oil, lavender oil, fennel oil, chamomile oil, perilla oil, spearmint oil and the like. In the present invention, the essential oil containing a terpene is preferably one or more selected from the group consisting of orange peel oil, orange oil, mentha oil, eucalyptus oil, lemon oil, ginger oil, clove oil, cinnamon oil, fennel oil, and perilla oil.

These terpenes may be used alone or in combination of two or more thereof.

In the present invention, the terpene is preferably one or more selected from the group consisting of menthol and mentha oil, particularly preferably 1-menthol.

The content of the terpene in the claimed film preparation of the present invention may be suitably selected depending on the type of a medicament, and from the standpoint of masking an unpleasant taste derived from a medicament (namely loxoprofen sodium hydrate or pitavastatin calcium), the content is 0.2 to 15% by mass, more preferably 0.5 to 10% by mass, still more preferably 1 to 5% by mass, particularly preferably 1 to 3% by mass with respect to the whole film preparation. In a particularly preferred embodiment, the content of the terpene in the medicament-containing layer is preferably 1 to 5% by mass, more preferably 1.5 to 4% by mass.

Further, the content mass ratio of the medicament to the terpene (medicament/terpene) is preferably 0.5 to 30, particularly preferably 1 to 25 from the standpoint of further reducing a bad sensation when taking the film preparation.

In the present invention, in order to improve film formability, it is preferred to incorporate a film-forming agent into one or both of the medicament-containing layer and the coating layer, and it is preferred to incorporate a film-forming agent into both of the medicament-containing layer and the coating layer (that is, "film preparation characterized by including coating layers containing a film-forming agent (but containing no terpene) on both sides of a medicament-containing layer containing a medicament having an unpleasant taste (namely loxoprofen sodium hydrate or pitavastatin calcium), a terpene, and a film-forming agent"). It should be noted that, in the case where the film-forming agent is incorporated into both of the medicament-containing layer and the coating layer, the types and amounts of the film-forming agent to be incorporated into the layers may be identical to or different from each other.

In the present invention, the type of the "film-forming agent" is not particularly limited as long as it has film formability. Specific examples of the film-forming agent include: alkylcelluloses such as methylcellulose and ethylcellulose; alginic acid or salts thereof such as sodium alginate; carrageenan; carboxyalkylcelluloses such as sodium carboxymethylcellulose, calcium carboxymethylcellulose, potassium carboxymethylcellulose, carboxymethylcellulose, and carboxymethylethylcellulose; xanthan gum; hydroxyalkylcelluloses such as hydroxymethylcellulose, hydroxyethylcellulose, hydroxypropylcellulose, and hypromellose (hydroxypropylmethylcellulose); hydroxyalkylcellulose phthalate such as hydroxypropylmethylcellulose phthalate; pullulan; polyvinyl acetate; polyvinyl acetate phthalate; polyvinyl alcohol; and polyvinyl pyrrolidone, and the like. The film-forming agent is preferably one or a combination of two or more selected from these materials.

In the present invention, the film-forming agent is preferably a film-forming agent having property of forming a film when an aqueous solution of the agent is dried, more preferably one or more selected from the group consisting of: alginic acid or salts thereof such as sodium alginate; carboxyalkylcelluloses such as sodium carboxymethylcellulose, calcium carboxymethylcellulose, potassium carboxymethylcellulose, carboxymethylcellulose, and carboxymethylethylcellulose; hydroxyalkylcelluloses such as hydroxymethylcellulose, hydroxyethylcellulose, hydroxypropylcellulose, and hypromellose; pullulan; polyvinyl alcohol; and polyvinyl pyrrolidone, still more preferably one or more selected from the group consisting of sodium alginate, carboxymethylcellulose, potassium carboxymethylcellulose, calcium carboxymethylcellulose, sodium carboxymethylcellulose, hydroxyethylcellulose, hydroxypropylcellulose, hypromellose, pullulan, polyvinyl alcohol, and polyvinyl pyrrolidone, yet still more preferably one or more selected from the group consisting of hypromellose and hydroxypropylcellulose. It is particularly preferred to incorporate hypromellose into the coating layer and incorporate hydroxypropylcellulose into the medicament-containing layer.

Hypromellose used herein means a mixed ether of methyl and hydroxypropyl of cellulose and may be produced by known methods . Further, as hypromellose, commercially available products (e.g., those produced by Shin-Etsu Chemical Co., Ltd., Dow Chemical Japan Ltd., Matsumoto Yushi-Seiyaku Co., Ltd., and the like) may be used. The substitution degree of a methoxy group and a hydroxypropoxy group in hypromellose is not particularly limited. Hypromellose with a desired substitution degree can be obtained by presetting the substitution degree before etherifying cellulose. In the present invention, hypromellose contains preferably 10 to 50%, more preferably 16.5 to 30%, particularly preferably 25 to 30% of a methoxy group and contains preferably 2 to 35%, more preferably 4 to 32%, particularly preferably 4 to 20% of a hydroxypropoxy group. Of those, hypromellose containing 16.5 to 30% of a methoxy group and 4 to 32% of a hydroxypropoxy group is particularly preferred. Further, hypromellose containing 25 to 30% of a methoxy group and 4 to 20% of a hydroxypropoxy group is more preferred. Among the commercially available products, Hypromellose 1828, Hypromellose 2208, Hypromellose 2906, and Hypromellose 2910 are preferred. It should be noted that the viscosity of hypromellose is not particularly limited, but for example, the kinematic viscosity of a 2% aqueous solution at 20°C (Japanese Pharmacopoeia 15th Edition) is preferably approx. 6 mPa·s.

On the other hand, hydroxypropylcellulose means hydroxypropyl ether of cellulose and may be produced by known methods . As hydroxypropylcellulose, commercially available products (e.g. , those produced by San-Ei Gen F.F.I, Inc or Nippon Soda Co., Ltd.) may be used. The substitution degree in hydroxypropylcellulose is not particularly limited. Hydroxypropylcellulose with a desired substitution degree can be obtained by presetting the substitution degree before etherifying cellulose. In the present invention, hydroxypropylcellulose contains preferably 50 to 80%, more preferably 53.4 to 77.5% of a hydroxypropoxy group. It should be noted that the viscosity of hydroxypropylcellulose is not particularly limited, but for example, the kinematic viscosity of a 2% aqueous solution at 20°C (Japanese Pharmacopoeia 15th Edition) is preferably 2.0 to 2.9 mPa·s.

The content of the film-forming agent in the film preparation of the present invention is not particularly limited, but is preferably 25 to 95% by mass, more preferably 30 to 80% by mass, particularly preferably 35 to 65% by mass with respect to the whole film preparation. In a particularly preferred embodiment, the content of the film-forming agent in the coating layer is preferably 45 to 80% by mass, particularly preferably 50 to 75% by mass, and the content of the film-forming agent in the medicament-coating layer is preferably 20 to 70% by mass, particularly preferably 25 to 60% by mass.

Further, in the present invention, it is preferred to incorporate a plasticizer into one or both of the medicament-containing layer and the coating layer to impart appropriate flexibility to the film preparation. It should be noted that, in the case where a plasticizer is incorporated into both of the medicament-containing layer and the coating layer, the types and amounts of the plasticizer to be incorporated into the layers may be identical to or different from each other.

In the present invention, the "plasticizer" means a compound which is compatible with the film-forming agent and imparts flexibility to the film-forming agent. The plasticizer is not particularly limited so long as the material of the plasticizer has such plasticizing ability. Examples thereof include glycerin, sesame oil, sorbitol, castor oil, propylene glycol, polyoxyethylene polyoxypropylene glycol, polysorbate 80 (polyoxyethylene (20) sorbitan oleic acid ester), and polyethylene glycol (e.g. , macrogol 400 (**n** (polymerization degree of oxyethylene units)=7 to 9, hereinafter, the polymerization degree of oxyethylene units is similarly represented by **n**), macrogol 600 (**n**=11 to 13), macrogol 1500 (an equivalent mixture of **n**=5 to 6 with **n**=28 to 36), macrogol 4000 (**n**=59 to 84), macrogol 6000 (**n**=165 to 210)), and the like. As the plasticizer, one or a combination of two or more selected from these materials is preferred, and one or more selected from the group consisting of glycerin, propylene glycol, and macrogol 400 are more preferred.

The content of the plasticizer in the film preparation of the present invention is not particularly limited, but is preferably 1 to 20% by mass, more preferably 3 to 15% by mass, particularly preferably 5 to 10% by mass with respect to the whole film preparation. In a particularly preferred embodiment, the content of the plasticizer in each of the coating layer and the medicament-containing layer is preferably 3 to 10% by mass, particularly preferably 4 to 10% by mass.

Further, one or two or more of pharmaceutical additives usually used may be used in the film preparation of the present invention, if necessary. Examples of the pharmaceutical additives include, but are not limited to, a disintegrant, a diluent, a poorly water-soluble polymeric substance, a coloring agent, an antioxidant, a flavoring agent, and a aromatic agent.

Examples of the disintegrant include starch, sucrose esters of fatty acids, gelatin, sodium bicarbonate, dextrin, dehydroacetic acid and salts thereof, povidone, and polyoxyethylene hydrogenated castor oil.

Examples of the diluent include: inorganic diluents such as titanium oxide, magnesium hydroxide-aluminium hydroxide co-precipitate, magnesium hydroxide, aluminium silicate, silicon dioxide, anhydrous sodium sulfate, anhydrous dibasic calcium phosphate, sodium chloride, amorphous silicon oxide hydrate, magnesium aluminosilicate, calcium silicate, magnesium silicate, light anhydrous silicic acid, heavy anhydrous silicic acid, magnesium oxide, calcium chloride, calcium sulfate, calcium monohydrogen phosphate, dibasic calcium phosphate, dibasic sodium phosphate, monobasic potassium phosphate, monobasic calcium phosphate, and sodium dihydrogen phosphate; and organic diluents such as maltose syrup powder, starch, fructose, caramel, agar, xylitol, paraffin, cellulose, sucrose, fructose, maltose, lactose, white soft sugar, glucose, pullulan, maltitol, hydrogenated maltose starch syrup, powdered hydrogenated maltose starch syrup, erythritol, xylitol, mannitol, lactitol, trehalose, hydrogenated palatinose, and maltose.

Examples of the poorly water-soluble polymeric substance include a carboxyvinyl polymer, an aminoalkyl methacrylate copolymer, and the like.

Examples of the coloring agent include yellow ferric oxide, brown iron oxide, caramel, black iron oxide, titanium oxide, red ferric oxide, a tar dye, an aluminium lake dye, sodium copper chlorophyllin, and the like.

Examples of the antioxidant include ascorbic acid, sodium bisulfite, sodium sulfite, disodiumedetate, erythorbic acid, tocopherol acetate, dibutylhydroxytoluene, natural vitamin E, tocopherol, butylhydroxyanisole, and the like.

Examples of the flavoring agent include an acidulant such as ascorbic acid, tartaric acid, citric acid, malic acid, and salts thereof, a sweetener such as aspartame, stevia, sucralose, glycyrrhizinic acid, thaumatin, acesulfame potassium, saccharin, and saccharin sodium, and the like.

It should be noted that, when the disintegrant is added, the disintegrant may be added in the range of 1 to 8% by mass with respect to the whole film preparation.

When the diluent is added, the diluent may be added in the range of 5 to 60% by mass with respect to the whole film preparation.

When the poorly water-soluble polymeric substance is added, the poorly water-soluble polymeric substance may be added in the range of 1 to 12.5% by mass with respect to the whole film preparation. When the coloring agent is added, the coloring agent may be added in the range of 0.05 to 10% by mass with respect to the whole film preparation. When the antioxidant is added, the antioxidant may be added in the range of 0.1 to 5% by mass with respect to the whole film preparation. When the flavoring agent is added, the flavoring agent may be added in the range of 1 to 10% by mass with respect to the whole film preparation. When the aromatic agent is added, the aromatic agent may be added in the range of 0.01 to 0.1% by mass with respect to the whole film preparation.

Specific forms of the film preparation of the present invention (such as the number and sizes of layers) are not particularly limited so long as the preparation includes coating layers on both sides of a medicament-containing layer.

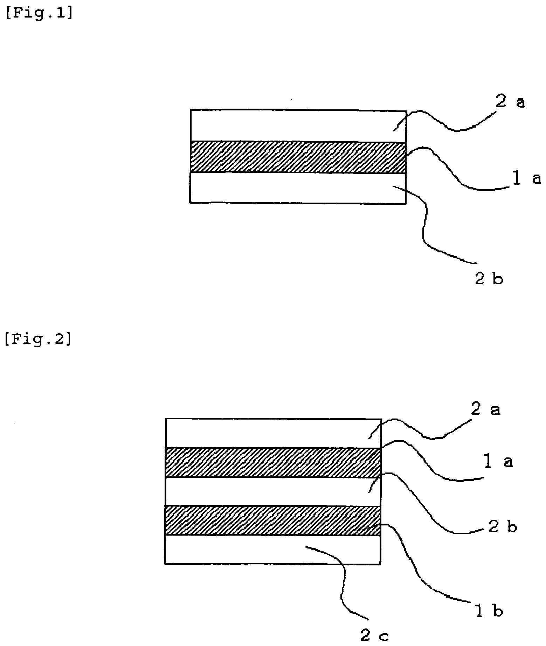

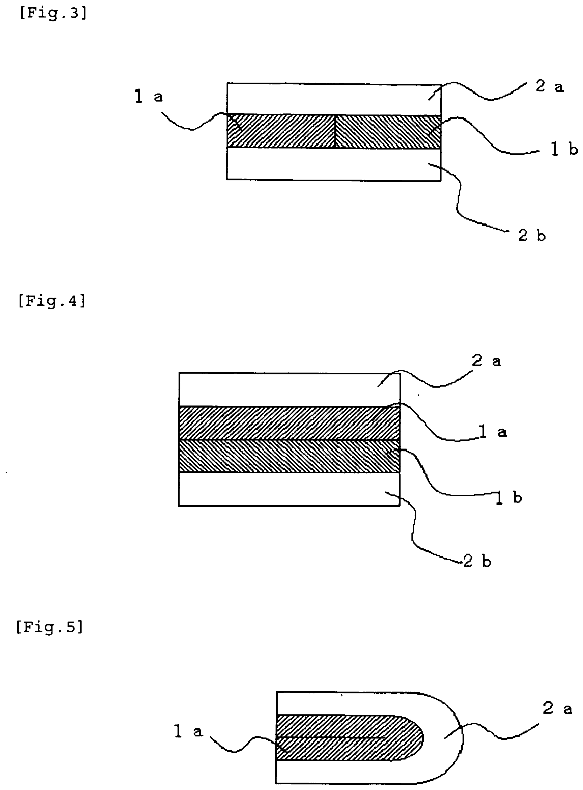

Specifically, the film preparation of the claimed present invention may have a form including coating layers laminated on both sides of a medicament-containing layer, such as a laminated three-layer form including the coating layer, the medicament-containing layer, and the coating layer, laminated in this order (FIG. 1 illustrates this form); or a laminated five-layer form including the coating layer, the medicament-containing layer, the coating layer, the medicament-containing layer, and the coating layer, laminated in this order (FIG. 2 illustrates this form) . It should be noted that, in the case where the film preparation includes a plurality of medicament-containing layers and/or coating layers, the types and amounts of components in the layers may be identical to or different from each other. The medicament-containing layers which contain different of medicaments may be provided adjacent to each other, and the coating layers may be laminated on both sides of the medicament-containing layers (FIG. 3 illustrates this form). The medicament-containing layers which contain different kinds of medicaments may be laminated, and the coating layers may be laminated on both sides of the medicament-containing layers (FIG. 4 illustrates this form).

Further, in the present invention, when layers of the same type are laminated adjacent to each other, they are integrated to each other to exert the same function. In the present invention, these layers are therefore regarded as substantially one layer (for example, a laminated four-layer structure including the coating layer, the medicament-containing layer, the medicament-containing layer, and the coating layer is taken as synonymous with a laminated three-layer structure including the coating layer, the medicament-containing layer, and the coating layer). Therefore, also in a form obtained by laminating the coating layer only on one side of the medicament-containing layer and folding the resultant into two such that the medicament-containing layer is provided inside (FIG. 5 illustrates this form), the medicament-containing layer folded inside is regarded as substantially one layer, and the coating layer is located on both sides of the medicament-containing layer. Therefore, the form is included in the film preparation of the present invention.

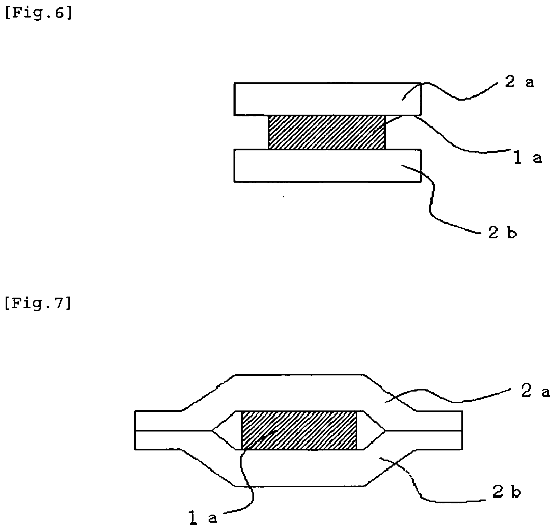

In addition, the sizes (areas) of the medicament-containing layer and the coating layer may be identical to or different from each other. The film preparation of the present invention includes a form including the medicament-containing layer which is smaller than the coating layers (FIG. 6 illustrates this form) and a form including the medicament-containing layer which is smaller than the coating layers and is enclosed with the coating layers (FIG. 7 illustrates this form).

It should be noted that the form of the film preparation of the present invention is not limited to the specific forms illustrated in FIGS. 1 to 7, and various modifications may be made without departing from the purpose of the present invention.

The thickness of the whole film preparation is not particularly limited, but is preferably 30 to 300 µm, particularly preferably 30 to 200 µm. In this case, the thickness of the coating layer is preferably 5 to 100 µm, particularly preferably 10 to 50 µm. The thickness of the medicament-containing layer is preferably 10 to 200 µm, particularly preferably 10 to 100 µm. With this, the preparation can be rapidly dissolved in the oral cavity. Further, the size of the film preparation of the present invention is not particularly limited so long as it is easily taken. For example, a size of approx. 0.5 to 10 cm² is preferred. The shape is not particularly limited either so long as it is easily taken. For example, a rectangular shape, a circular shape, or an elliptical shape may be suitably selected.

The film preparation of the present invention may be suitably prepared by common or known methods. For example, the film preparation of the present invention may be prepared by laminating a first coating layer on a release film made of polyethylene terephthalate (PET) or the like, laminating a medicament-containing layer thereon, and then laminating a second coating layer on the medicament-containing layer. Alternatively, the film preparation of the present invention may also be prepared by separately preparing an interim product obtained by laminating a first coating layer on a release film and further laminating a medicament-containing layer thereon, and an interim product prepared by laminating a second coating layer on a release film and further laminating a medicament-containing layer thereon, and then attaching the medicament-containing layers of both interim products so that they are opposed to each other and laminating them by pressure. It should be noted that the film-forming agents in the first coating layer and the second coating layer may be identical to or different from each other.

The film preparation of the present invention is preferably administered orally (that is, the preparation is preferably "film preparation for oral administration"). In particular, the film preparation of the present invention is particularly preferably produced as a film preparation which is dissolved in the oral cavity because a bad sensation when taking the film preparation can be reduced, and the preparation can be dissolved only with moisture in the oral cavity and hence can be easily taken. It should be noted that the film preparation of the present invention, produced as a film preparation which is dissolved in the oral cavity, can be suitably prepared by common or known methods.

### Examples

Hereinafter, the present invention is more specifically described with reference to the examples and the like. However, the scope of the present invention is not limited to the examples described below.

### (Example 1)

### - outside the scope of the claims-

50 g of powdered hydrogenated maltose starch syrup and 40 g of macrogol 400 were dissolved in 500 g of water. Then, to this solution was added a solution obtained by dispersing 50 g of titanium oxide in 750 g of anhydrous ethanol. Then, 360 g of hypromellose was added to the resultant solution to obtain a solution for preparing a coating layer.

27 g of loperamide hydrochloride, 64.8 g of macrogol 400, 256.5 g of powdered hydrogenated maltose starch syrup, 54 g of saccharin sodium, and 27 g of 1-menthol were dissolved in a mixture solution of 810 g of water and 810 g of anhydrous ethanol. To this mixture solution were added 380.7 g of hydroxypropylcellulose and 1.35 g of a aromatic agent to obtain a solution for preparing a medicament-containing layer.

The solution for preparing a coating layer was uniformly applied onto a PET film and then dried with hot air to form a coating layer with a mass of 5 mg per area of 2.72 cm². The solution for preparing a medicament-containing layer was uniformly applied onto the upper side of the coating layer and then dried with hot air to form a medicament-containing layer with a mass of 7.5125 mg per area of 2.72 cm² as Interim Product 1.

Two sets of Interim Product 1 were prepared, attached so that the medicament-containing layers were opposed to each other, and bonded by pressure, to obtain Interim Product 2 in which the coating layer, the medicament-containing layer, and the coating layer were laminated in this order between the two PET films.

One PET film of Interim Product 2 was peeled off, the product was cut into an area of 2.72 cm², and the other PET film was peeled off to obtain the film preparation of Example 1.

### (Comparative Example 1)

### - outside the scope of the claims-

50 g of powdered hydrogenated maltose starch syrup and 40 g of macrogol 400 were dissolved in 500 g of water. Then, to this solution were added a solution obtained by dissolving 25 g of 1-menthol in 250 g of anhydrous ethanol and a solution obtained by dispersing 50 g of titanium oxide in 500 g of anhydrous ethanol. Then, 335 g of hypromellose was added to the resultant solution to obtain a solution for preparing a coating layer.

27 g of loperamide hydrochloride, 64.8 g of macrogol 400, 256.5 g of powdered hydrogenated maltose starch syrup, and 54 g of saccharin sodium were dissolved in a mixture solution of 810 g of water and 810 g of anhydrous ethanol. To this mixture solution were added 407.7 g of hydroxypropylcellulose and 1.35 g of a aromatic agent to obtain a solution for preparing a medicament-containing layer.

The solution for preparing a coating layer was uniformly applied onto a PET film and then dried with hot air to form a coating layer with a mass of 5 mg per area of 2.72 cm². The solution for preparing a medicament-containing layer was uniformly applied onto the upper side of the coating layer and then dried with hot air to form a medicament-containing layer with a mass of 7.5125 mg per area of 2.72 cm² as Interim Product 1.

Two sets of Interim Product 1 were prepared, attached so that the medicament-containing layers were opposed to each other, and bonded by pressure, to obtain Interim Product 2 in which the coating layer, the medicament-containing layer, and the coating layer were laminated in this order between the two PET films.

One PET film of Interim Product 2 was peeled off, the product was cut into an area of 2.72 cm², and the other PET film was peeled off to obtain a film preparation of Comparative Example 1.

### (Comparative example 2)

### - outside the scope of the claims-

50 g of powdered hydrogenated maltose starch syrup and 40 g of macrogol 400 were dissolved in 500 g of water. Then, to this solution were added a solution obtained by dissolving 25 g of 1-menthol in 250 g of anhydrous ethanol and a solution obtained by dispersing 50 g of titanium oxide in 500 g of anhydrous ethanol. Then, 335 g of hypromellose was added to the resultant solution to obtain a solution for preparing a coating layer.

27 g of loperamide hydrochloride, 64.8 g of macrogol 400, 256.5 g of powdered hydrogenated maltose starch syrup, 54 g of saccharin sodium, and 27 g of 1-menthol were dissolved in a mixture solution of 810 g of water and 810 g of anhydrous ethanol. 380.7 g of hydroxypropylcellulose and 1.35 g of a aromatic agent were added to this mixture solution to obtain a solution for preparing a medicament-containing layer.

The solution for preparing a coating layer was uniformly applied onto a PET film and then dried with hot air to form a coating layer with a mass of 5 mg per area of 2.72 cm². The solution for preparing a medicament-containing layer was uniformly applied onto the upper side of the coating layer and then dried with hot air to form a medicament-containing layer with a mass of 7.5125 mg per area of 2.72 cm² as Interim Product 1.

Two sets of Interim Product 1 were prepared, attached so that the medicament-containing layers were opposed to each other, and bonded by pressure, to obtain Interim Product 2 in which the coating layer, the medicament-containing layer, and the coating layer were laminated in this order between the two PET films.

One PET film of Interim Product 2 was peeled off, the product was cut into an area of 2.72 cm², and the other PET film was peeled off to obtain a film preparation of Comparative Example 2.

### (Test Example 1) Bitter taste masking test

The film preparations obtained in Example 1, Comparative Example 1, and Comparative Example 2 were evaluated for sensation when these preparations were taken. A sensory evaluation was conducted, in which the film preparations were evaluated by six panelists for sensation when these preparations were taken (bitter taste). The evaluation criteria are as follows: "a bitter taste is virtuallyunnoticeable" given 2 points; "abitter taste is slightly noticeable" given 1 point; and "a bitter taste is noticeable and the taste is bad" given 0 points. Accordingly, a higher total score indicates that a bitter taste is more masked. The total scores are shown in Table 1, with the composition of each film preparation (amounts (mg) of the component per film preparation).

### - outside the scope of the claims-

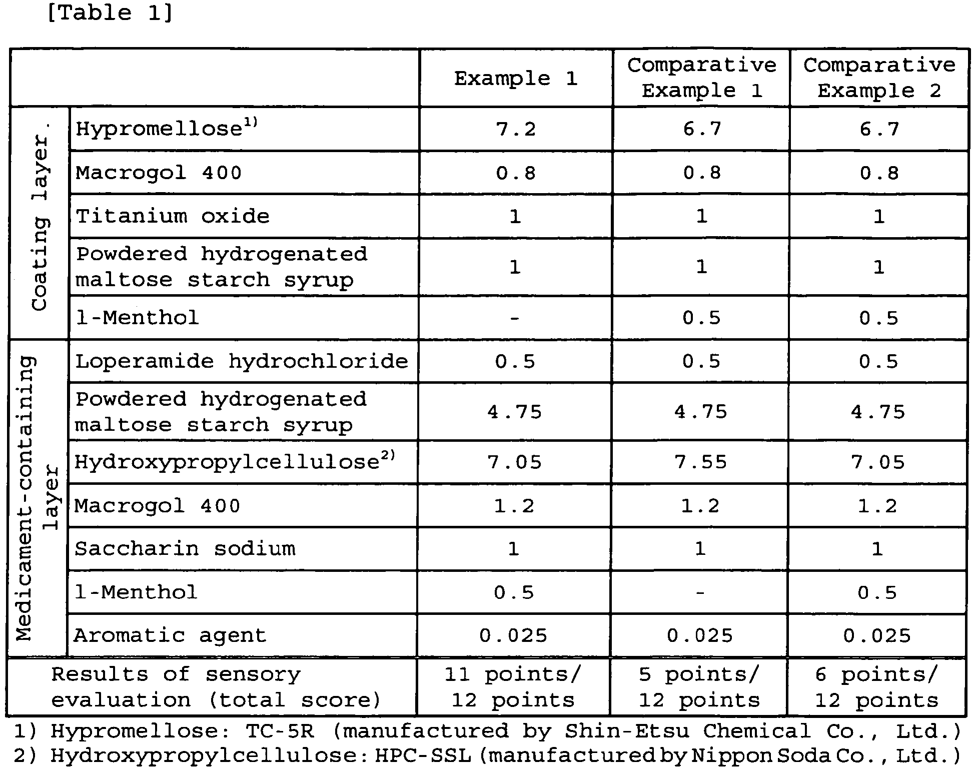

As evident from Table 1, it was confirmed that, the film preparation of the present invention (Example 1) with a medicament-containing layer containing loperamide hydrochloride having a strong bitter taste together with menthol, which was sandwiched between coating layers not containing menthol, could mask the bad taste of loperamide hydrochloride and a film preparation with an easy-to-take could be produced.

On the other hand, the film preparation of Comparative Example 1 with a medicament-containing layer containing loperamide hydrochloride but not menthol, which was sandwiched between coating layers containing menthol, could not mask the bad taste of loperamide hydrochloride. Further, the film preparation of Comparative Example 2 with a medicament-containing layer containing loperamide hydrochloride and menthol, which was sandwiched between coating layers containing menthol, could not mask the unpleasant taste loperamide hydrochloride either.

Accordingly, it was found that masking of the unpleasant taste of loperamide hydrochloride could not be achieved simply by adding menthol, and masking of the bad taste could be achieved by adding menthol to only a medicament-containing layer.

Further, when the film preparation of the present invention (Example 1) was taken without water, it was dissolved in the oral cavity within 30 seconds. It was therefore confirmed that the fast-acting property of the film preparation could be expected.

### (Example 2)

432 g of water and 432 g of anhydrous ethanol were mixed, and 24 g of macrogol 400 and 30 g of powdered hydrogenated maltose starch syrup were dissolved, followed by dispersion of 30 g of titanium oxide. 216 g of hypromellose was dissolved in the resultant solution to obtain a solution for preparing a coating layer.

432 g of water and 432 g of anhydrous ethanol were mixed, and 340.5 g of loxoprofen sodium hydrate, 36 g of macrogol 400, 142.5 g of powdered hydrogenated maltose starch syrup, 30 g of saccharin sodium, and 15 g of 1-menthol were dissolved. 211.5 g of hydroxypropylcellulose was dissolved in the resultant solution to obtain a solution for preparing a medicament-containing layer.

The solution for preparing a coating layer was uniformly applied onto a PET film and then dried with hot air to form a coating layer with a mass of 5 mg per area of 2.8 cm². The solution for preparing a medicament-containing layer was uniformly applied onto the upper side of the coating layer and then dried with hot air to form a medicament-containing layer with a mass of 12.925 mg per area of 2.8 cm² as Interim Product 1.

Two sets of Interim Product 1 were prepared, attached so that the medicament-containing layers were opposed to each other, and bonded by pressure, to obtain Interim Product 2 in which the coating layer, the medicament-containing layer, and the coating layer were laminated in this order between the two PET films.

Interim Product 2 was cut into an area of 2.8 cm², and the PET film was peeled off to obtain a film preparation of Example 2.

### (Comparative Example 3)

432 g of water and 432 g of anhydrous ethanol were mixed, and 24 g of macrogol 400, 30 g of powdered hydrogenated maltose starch syrup, and 15 g of 1-menthol were dissolved, followed by dispersion of 30 g of titanium oxide. 216 g of hypromellose was dissolved in the resultant solution to obtain a solution for preparing a coating layer.

432 g of water and 432 g of anhydrous ethanol were mixed, and 340.5 g of loxoprofen sodium hydrate, 36 g of macrogol 400, 142.5 g of powdered hydrogenated maltose starch syrup, and 30 g of saccharin sodium were dissolved. 211.5 g of hydroxypropylcellulose was dissolved in the resultant solution to obtain a solution for preparing a medicament-containing layer.

The solution for preparing a coating layer was uniformly applied onto a PET film and then dried with hot air to form a coating layer with a mass of 5.25 mg per area of 2.8 cm². The solution for preparing a medicament-containing layer was uniformly applied onto the upper side of the coating layer and then dried with hot air to form a medicament-containing layer with a mass of 12.675 mg per area of 2.8 cm² as Interim Product 1.

Two sets of Interim Product 1 were prepared, attached so that the medicament-containing layers were opposed to each other, and bonded by pressure, to obtain Interim Product 2 in which the coating layer, the medicament-containing layer, and the coating layer were laminated in this order between the two PET films.

Interim Product 2 was cut into an area of 2.8 cm², and the PET film was peeled off to obtain a film preparation of Comparative Example 3.

### (Comparative Example 4)

432 g of water and 432 g of anhydrous ethanol were mixed, and 24 g of macrogol 400, 30 g of powdered hydrogenated maltose starch syrup, and 15 g of 1-methol were dissolved, followed by dispersion of 30 g of titanium oxide. 216 g of hypromellose was dissolved in the resultant solution to obtain a solution for preparing a coating layer.

432 g of water and 432 g of anhydrous ethanol were mixed, and 340.5 g of loxoprofen sodium hydrate, 36 g of macrogol 400, 142.5 g of powdered hydrogenated maltose starch syrup, 30 g of saccharin sodium, and 15 g of 1-menthol were dissolved. 211.5 g of hydroxypropylcellulose was dissolved in the resultant solution to obtain a solution for preparing a medicament-containing layer.

The solution for preparing a coating layer was uniformly applied onto a PET film and then dried with hot air to form a coating layer with a mass of 5.25 mg per area of 2.8 cm². The solution for preparing a medicament-containing layer was uniformly applied onto the upper side of the coating layer and then dried with hot air to form a medicament-containing layer with a mass of 12.925 mg per area of 2.8 cm² as Interim Product 1.

Two sets of Interim Product 1 were prepared, attached so that the medicament-containing layers were opposed to each other, and bonded by pressure, to obtain Interim Product 2 in which the coating layer, the medicament-containing layer, and the coating layer were laminated in this order between the two PET films.

Interim Product 2 was cut into an area of 2.8 cm², and the PET film was peeled off to obtain a film preparation of Comparative Example 4.

### (Test Example 2) Bitter taste masking test

The film preparations obtained in Example 2, Comparative Example 3, and Comparative Example 4 were evaluated for sensation when these preparations were taken by a sensory evaluation in the same manner as in Test Example 1 except that the number of panelists was five. The total scores are shown in Table 2, with the composition of each film preparation (amounts (mg) of components per film preparation).

**[Table 2]**

| | | Example 2 | Comparative Example 3 | Comparative Example 4 |
|---|---|---|---|---|
| Coating layer | Hypromellose¹⁾ | 7.2 | 7.2 | 7.2 |
| | Macrogol 400 | 0.8 | 0.8 | 0.8 |
| | Titanium oxide | 1 | 1 | 1 |
| | Powdered hydrogenated maltose starch syrup | 1 | 1 | 1 |
| | 1-Menthol | - | 0.5 | 0.5 |
| Medicament -containin g layer | Loxoprofen sodium hydrate³⁾ | 11.35 | 11.35 | 11.35 |
| | Powdered hydrogenated maltose starch syrup | 4.75 | 4.75 | 4.75 |
| | Hydroxypropylcellulose²⁾ | 7.05 | 7.05 | 7.05 |
| | Macrogol 400 | 1.2 | 1.2 | 1.2 |
| | Saccharin sodium | 1 | 1 | 1 |
| | 1-Menthol | 0.5 | - | 0.5 |
| Results of sensory evaluation (total score) | | 9 points/10 points | 3 points/10 points | 1 point/10 points |

| | | | | |
|---|---|---|---|---|
| 1) Hypromellose: TC-5R (manufactured by Shin-Etsu Chemical Co., Ltd.) 2) Hydroxypropylcellulose: HPC-SSL (manufactured by Nippon Soda Co., Ltd.) 3) 10 mg in terms of anhydrous loxoprofen sodium | | | | |

As evident from Table 2, it was confirmed that, the film preparation of the present invention (Example 2) with a medicament-containing layer containing loxoprofen sodium hydrate having a bitter taste together with menthol, which was sandwiched between coating layers not containing menthol, could mask the bad taste of loxoprofen sodium hydrate, and a film preparation with an easy-to-take could be produced.

On the other hand, the film preparation of Comparative Example 3 with a medicament-containing layer containing loxoprofen sodium hydrate but not menthol, which was sandwiched between coating layers containing menthol, could not mask the unpleasant taste of loxoprofen sodium hydrate. Further, the film preparation of Comparative Example 4 with a medicament-containing layer containing loxoprofen sodium hydrate and menthol, which was sandwiched between coating layers containing menthol, could not mask the unpleasant taste of loxoprofen sodium hydrate either.

Accordingly, it was found that masking of the unpleasant taste of loxoprofen sodium hydrate could not be achieved simply by adding menthol, and masking of the unpleasant taste could be achieved by adding menthol to only a medicament-containing layer.

### (Example 3)

480 g of water and 480 g of anhydrous ethanol were mixed, and 30 g of macrogol 400, 120 g of an aminoalkyl methacrylate copolymer E, and 30 g of trehalose were dissolved, followed by dispersion of 30 g of titanium oxide and 3 g of red ferric oxide. 240 g of hypromellose was dissolved in the resultant solution to obtain a solution for preparing a coating layer.

600 g of water and 600 g of anhydrous ethanol were mixed, and 30 g of macrogol 400, 60 g of calcium chloride, 30 g of sucralose, and 15 g of 1-menthol were dissolved, followed by dispersion of 60 g of pitavastatin calcium and 30 g of magnesium oxide. 300 g of hydroxypropylcellulose was dissolved in the resultant solution to obtain a solution for preparing a medicament-containing layer.

The solution for preparing a coating layer was uniformly applied onto a PET film and then dried with hot air to form a coating layer with a mass of 7.55 mg per area of 2.8 cm². The solution for preparing a medicament-containing layer was uniformly applied onto the upper side of the coating layer and then dried with hot air to form a medicament-containing layer with a mass of 8.75 mg per area of 2.8 cm² as Interim Product 1.

Two sets of Interim Product 1 were prepared, attached so that the medicament-containing layers were opposed to each other, and bonded by pressure, to obtain Interim Product 2 in which the coating layer, the medicament-containing layer, and the coating layer were laminated in this order between the two PET films.

Interim Product 2 was cut into an area of 2.8 cm², and the PET film was peeled off to obtain a film preparation of Example 3 .

### (Test Example 3) Bitter taste masking test

The film preparation obtained in Example 3 was evaluated for sensation when it was taken by a sensory evaluation in the same manner as in Test Example 1 except that the number of panelists was five. The total score is shown in Table 3, with the composition of film preparation (amounts (mg) of components per film preparation).

**[Table 3]**

| | | Example 3 |
|---|---|---|
| Coating layer | Hypromellose¹⁾ | 8 |
| | Aminoalkyl methacrylate copolymer E | 4 |
| | Titanium oxide | 1 |
| | Red Ferric oxide | 0.1 |
| | Macrogol 400 | 1 |
| | Trehalose | 1 |
| Medicament-containing layer | Pitavastatin calcium | 2 |
| | Hydroxypropylcellulose²⁾ | 10 |
| | Sucralose | 1 |
| | 1-Menthol | 0.5 |
| | Macrogol 400 | 1 |
| | Magnesium oxide | 1 |
| | Calcium chloride | 2 |
| Results of sensory evaluation (total score) | | 10 points/10 points |

| | | |
|---|---|---|
| 1) Hypromellose: TC-5R (manufactured by Shin-Etsu Chemical Co., Ltd.) 2) Hydroxypropylcellulose: HPC-SSL (manufactured by Nippon Soda Co., Ltd.) | | |

As evident from Table 3, it was confirmed that, the film preparation of the present invention (Example 3) with a medicament-containing layer containing pitavastatin calcium having a bitter taste together with menthol, which was sandwiched between coating layers not containing menthol, could mask the unpleasant taste of pitavastatin calcium and a film preparation with an easy-to-take could be produced.

### (Example 4)

### - outside the scope of the claims-

480 g of water and 480 g of anhydrous ethanol were mixed, and 30 g of macrogol 400, and 120 g of an aminoalkyl methacrylate copolymer E, and 30 g of trehalose were dissolved, followed by dispersion of 30 g of titanium oxide. 240 g of hypromellose was dissolved in the resultant solution to obtain a solution for preparing a coating layer.

30 g of macrogol 400, 30 g of sucralose, and 15 g of 1-menthol were dissolved in 900 g of anhydrous ethanol, followed by dispersion of 300 g of famotidine. 300 g of hydroxypropylcellulose was dissolved in the resultant solution to obtain a solution for preparing a medicament-containing layer.

The solution for preparing a coating layer was uniformly applied onto a PET film and then dried with hot air to form a coating layer with a mass of 7.5 mg per area of 2.8 cm². The solution for preparing a medicament-containing layer was uniformly applied onto the upper side of the coating layer and then dried with hot air to form a medicament-containing layer with a mass of 11.25 mg per area of 2.8 cm² as Interim Product 1.

Two sets of Interim Product 1 were prepared, attached so that the medicament-containing layers were opposed to each other, and bonded by pressure, to obtain Interim Product 2 in which the coating layer, the medicament-containing layer, and the coating layer were laminated in this order between the two PET films.

Interim Product 2 was cut into an area of 2.8 cm², and the PET film was peeled off to obtain a film preparation of Example 4.

### (Test Example 4) Bitter taste masking test

The film preparation obtained in Example 4 was evaluated for sensation when it was taken by a sensory evaluation in the same manner as in Test Example 1 except that the number of panelists was five. The total score is shown in Table 4, with the composition of film preparation (amounts (mg) of components per film preparation).

### - outside the scope of the claims-

**[Table 4]**

| | | Example 4 |
|---|---|---|
| Coating layer | Hypromellose¹⁾ | 8 |
| | Aminoalkyl methacrylate copolymer E | 4 |
| | Titanium oxide | 1 |
| | Macrogol 400 | 1 |
| | Trehalose | 1 |
| Medicament-containing layer | Famotidine | 10 |
| | Hydroxypropylcellulose²⁾ | 10 |
| | Sucralose | 1 |
| | 1-Menthol | 0.5 |
| | Macrogol 400 | 1 |
| Results of sensory evaluation (total score) | | 8 points/10 points |

| | | |
|---|---|---|
| 1) Hypromellose: TC-5R (manufactured by Shin-Etsu Chemical Co., Ltd.) 2) Hydroxypropylcellulose: HPC-SSL (manufactured by Nippon Soda Co., Ltd.) | | |

As evident from Table 4, it was confirmed that, the film preparation of the present invention (Example 4) with a medicament-containing layer containing famotidine having a bitter taste together with menthol, which was sandwiched between coating layers not containing menthol, could mask the unpleasant taste of famotidine, and a film preparation with an easy-to-take could be produced.

The results of Test Examples 1 to 4 show that, in the film preparations including the coating layers not containing terpene formed on both sides of the medicament-containing layer containing a medicament having an unpleasant taste such as a bitter taste, and a terpene, bad sensation when taking the film preparations can be reduced because an unpleasant taste derived from the medicament having an unpleasant taste is masked.

### (Production Example 1)

### - outside the scope of the claims-

A film preparation containing 10 mg of free paroxetine per area of 2.8 cm² (per film preparation) may be produced in the same manner as in Example 2 except for the use of 341.4 g of paroxetine hydrochloride hydrate instead of 340.5 g of loxoprofen sodium hydrate in Example 2.

### (Production Example 2)

### - outside the scope of the claims-

A film preparation containing 5 mg of free montelukast per area of 2.8 cm² (per film preparation) may be produced in the same manner as in Example 2 except for the use of 155.7 g of montelukast sodium instead of 340.5 g of loxoprofen sodium hydrate in Example 2.

### (Production Example 3)

### - outside the scope of the claims-

A film preparation containing 3 mg of donepezil hydrochloride per area of 2.8 cm² (per film preparation) may be produced in the same manner as in Example 2 except for the use of 90 g of donepezil hydrochloride instead of 340.5 g of loxoprofen sodium hydrate in Example 2.

### (Production Example 4)

### - outside the scope of the claims-

A film preparation containing 5 mg of free atorvastatin per area of 2.8 cm² (per film preparation) may be produced in the same manner as in Example 2 except for the use of 162.6 g of atorvastatin calcium hydrate instead of 340.5 g of loxoprofen sodium hydrate in Example 2.

### (Production Example 5)

### - outside the scope of the claims-

A film preparation containing 2.5 mg of free rosuvastatin per area of 2.8 cm² (per film preparation) may be produced in the same manner as in Example 2 except for the use of 78 g of rosuvastatin calcium instead of 340.5 g of loxoprofen sodium hydrate in Example 2.

### (Production Example 6) - outside the scope of the claims-

A film preparation containing 2 mg of candesartan cilexetil per area of 2.8 cm² (per film preparation) may be produced in the same manner as in Example 2 except for the use of 60 g of candesartan cilexetil instead of 340.5 g of loxoprofen sodium hydrate in Example 2.

### (Production Example 7)

### - outside the scope of the claims-

A film preparation containing 5 µg of limaprost alfadex per area of 2.8 cm² (per film preparation) may be produced in the same manner as in Example 2 except for the use of 533.5 mg of limaprost alfadex instead of 340.5 g of loxoprofen sodium hydrate in Example 2.

### (Production Example 8)

### - outside the scope of the claims-

A film preparation containing 2.5 mg of free amlodipine per area of 2.8 cm² (per film preparation) may be produced in the same manner as in Example 2 except for the use of 104.1 g of amlodipine besylate instead of 340.5 g of loxoprofen sodium hydrate in Example 2.

### (Production Example 9)

### - outside the scope of the claims-

A film preparation containing 0.125 mg of pramipexole hydrochloride hydrate per area of 2.8 cm² (per film preparation) may be produced in the same manner as in Example 2 except for the use of 3.75 g of pramipexole hydrochloride hydrate instead of 340.5 g of loxoprofen sodium hydrate in Example 2.

### (Production Example 10)

### - outside the scope of the claims-

A film preparation containing 5 mg of olanzapine per area of 2.8 cm² (per film preparation) may be produced in the same manner as in Example 2 except for the use of 150 g of olanzapine instead of 340.5 g of loxoprofen sodium hydrate in Example 2.

### (Production Example 11)

A film preparation containing 3 mg of aripiprazole per area of 2.8 cm² (per film preparation) may be produced in the same manner as in Example 2 except for the use of 90 g of aripiprazole instead of 340.5 g of loxoprofen sodium hydrate in Example 2.

### (Production Example 12)

### - outside the scope of the claims-

A film preparation containing 2 mg of silodosin per area of 2.8 cm² (per film preparation) may be produced in the same manner as in Example 2 except for the use of 60 g of silodosin instead of 340.5 g of loxoprofen sodium hydrate in Example 2.

### (Production Example 13)

### - outside the scope of the claims-

A film preparation containing 2.5 mg of solifenacin succinate per area of 2.8 cm² (per film preparation) may be produced in the same manner as in Example 2 except for the use of 75 g of solifenacin succinate instead of 340.5 g of loxoprofen sodium hydrate in Example 2.

### (Production Example 14)

### - outside the scope of the claims-

A film preparation containing 10 mg of sodium rabeprazole per area of 2.8 cm² (per film preparation) may be produced in the same manner as in Example 2 except for the use of 300 g of sodium rabeprazole instead of 340.5 g of loxoprofen sodium hydrate in Example 2.

### (Production Example 15)

### - outside the scope of the claims-

A film preparation containing 15 mg of free pioglitazone per area of 2.8 cm² (per film preparation) may be produced in the same manner as in Example 2 except for the use of 495.9 g of pioglitazone hydrochloride instead of 340.5 g of loxoprofen sodium hydrate in Example 2.

### (Production Example 16)

### - outside the scope of the claims-

A film preparation containing 10 mg of ezetimibe per area of 2.8 cm² (per film preparation) may be produced in the same manner as in Example 2 except for the use of 300 g of ezetimibe instead of 340.5 g of loxoprofen sodium hydrate in Example 2.

### (Production Example 17)

### - outside the scope of the claims-

A film preparation containing 2.5 mg of solifenacin succinate per area of 2.8 cm² (per film preparation) may be produced in the same manner as in Example 2 except for the use of 75 g of solifenacin succinate instead of 340.5 g of loxoprofen sodium hydrate in Example 2.

### (Production Example 18)

### - outside the scope of the claims-

A film preparation containing 5 mg of ebastine per area of 2.8 cm² (per film preparation) may be produced in the same manner as in Example 2 except for the use of 150 g of ebastine instead of 340.5 g of loxoprofen sodium hydrate in Example 2.

### (Production Example 19)

### - outside the scope of the claims-

A film preparation containing 1.25 mg of carvedilol per area of 2.8 cm² (per film preparation) may be produced in the same manner as in Example 2 except for the use of 37.5 g of carvedilol instead of 340.5 g of loxoprofen sodium hydrate in Example 2.

### (Production Example 20)

### - outside the scope of the claims-

A film preparation containing 8 mg of azelnidipine per area of 2.8 cm² (per film preparation) may be produced in the same manner as in Example 2 except for the use of 240 g of azelnidipine instead of 340.5 g of loxoprofen sodium hydrate in Example 2.

### (Production Example 21)

### - outside the scope of the claims-

A film preparation containing 5 mg of cetirizine hydrochloride per area of 2.8 cm² (per film preparation) may be produced in the same manner as in Example 2 except for the use of 150 g of cetirizine hydrochloride instead of 340.5 g of loxoprofen sodium hydrate in Example 2.

### (Production Example 22)

### - outside the scope of the claims-

A film preparation containing 2 mg of azelastine hydrochloride per area of 2.8 cm² (per film preparation) may be produced in the same manner as in Example 2 except for the use of 60 g of azelastine hydrochloride instead of 340.5 g of loxoprofen sodium hydrate in Example 2.

### (Production Example 23)

### - outside the scope of the claims-

A film preparation containing 5 mg of tiquizium bromide per area of 2.8 cm² (per film preparation) may be produced in the same manner as in Example 2 except for the use of 150 g of tiquizium bromide instead of 340.5 g of loxoprofen sodium hydrate in Example 2.

### (Production Example 24)

### - outside the scope of the claims-

A film preparation containing 10 mg of famotidine per area of 2.8 cm² (per film preparation) may be produced in the same manner as in Example 2 except for the use of 300 g of famotidine instead of 340.5 g of loxoprofen sodium hydrate in Example 2.

### (Production Example 25)

### - outside the scope of the claims-

A film preparation containing 2 mg of emedastine difumarate per area of 2.8 cm² (per film preparation) may be produced in the same manner as in Example 2 except for the use of 60 g of emedastine difumarate instead of 340.5 g of loxoprofen sodium hydrate in Example 2.

### (Production Example 26)

### - outside the scope of the claims-

A film preparation containing 5 mg of pemirolast potassium per area of 2.8 cm² (per film preparation) may be produced in the same manner as in Example 2 except for the use of 150 g of pemirolast potassium instead of 340.5 g of loxoprofen sodium hydrate in Example 2.

### (Production Example 27)

### - outside the scope of the claims-

A film preparation containing 10 mg of omeprazole per area of 2.8 cm² (per film preparation) may be produced in the same manner as in Example 2 except for the use of 300 g of omeprazole instead of 340.5 g of loxoprofen sodium hydrate in Example 2.

### (Production Example 28)

### - outside the scope of the claims-

A film preparation containing 15 mg of lansoprazole per area of 2.8 cm² (per film preparation) may be produced in the same manner as in Example 2 except for the use of 450 g of lansoprazole instead of 340.5 g of loxoprofen sodium hydrate in Example 2.

### (Production Example 29)

### - outside the scope of the claims-

A film preparation containing 0.25 µg of alfacalcidol per area of 2.8 cm² (per film preparation) may be produced in the same manner as in Example 2 except for the use of 7.5 mg of alfacalcidol instead of 340.5 g of loxoprofen sodium hydrate in Example 2.

### (Production Example 30)

### - outside the scope of the claims-

A film preparation containing 0.25 µg of calcitriol per area of 2.8 cm² (per film preparation) may be produced in the same manner as in Example 2 except for the use of 7.5 mg of calcitriol instead of 340.5 g of loxoprofen sodium hydrate in Example 2.

### (Production Example 31)

### - outside the scope of the claims-

A film preparation containing 5 mg of domperidone per area of 2.8 cm² (per film preparation) may be produced in the same manner as in Example 2 except for the use of 150 g of domperidone instead of 340.5 g of loxoprofen sodium hydrate in Example 2.

### (Production Example 32)

### - outside the scope of the claims-

A film preparation containing 10 mg of sodium rabeprazole per area of 2.8 cm² (per film preparation) may be produced in the same manner as in Example 2 except for the use of 300 g of sodium rabeprazole instead of 340.5 g of loxoprofen sodium hydrate in Example 2.

### (Production Example 33)

### - outside the scope of the claims-

A film preparation containing 5 mg of pravastatin sodium per area of 2.8 cm² (per film preparation) may be produced in the same manner as in Example 2 except for the use of 150 g of pravastatin sodium instead of 340.5 g of loxoprofen sodium hydrate in Example 2.

### (Production Example 34)

### - outside the scope of the claims-

A film preparation containing 5 mg of prasugrel hydrochloride per area of 2.8 cm² (per film preparation) may be produced in the same manner as in Example 2 except for the use of 150 g of prasugrel hydrochloride instead of 340.5 g of loxoprofen sodium hydrate in Example 2.

### (Production Example 35)

### - outside the scope of the claims-

A film preparation containing 30 mg of edoxaban tosylate hydrate per area of 2.8 cm² (per film preparation) may be produced in the same manner as in Example 2 except for the use of 900 g of edoxaban tosylate hydrate instead of 340.5 g of loxoprofen sodium hydrate in Example 2.

In the film preparations obtained in Production Examples 1 to 35 as well, the unpleasant tastes derived from the medicaments can be masked.

### Industrial Applicability

According to the present invention, it is possible to provide the claimed film preparation in which an unpleasant taste derived from a medicament is masked, and the preparation can be used in the pharmaceutical industry and the like.

### Reference Signs List

1a, 1b ··· medicament-containing layer
2a, 2b, 2c ··· coating layer

## Claims

1. A film preparation, comprising coating layers containing no terpene formed on both sides of a medicament-containing layer containing a medicament having an unpleasant taste, and a terpene, wherein the unpleasant taste is a bitter taste, a harsh taste, or an astringent taste, wherein the content of the terpene in the film preparation is 0.2 to 15% by mass with respect to the whole film preparation, and wherein the medicament having an unpleasant taste is loxoprofen sodium hydrate or pitavastatin calcium.

2. The film preparation according to claim 1, wherein the terpene is menthol.

3. A film preparation according to claim 1 or 2, wherein the medicament-containing layer or the coating layers, or the medicament-containing layer and the coating layers comprise a film-forming agent.

4. The film preparation according to claim 3, wherein the film-forming agent is hydroxypropylcellulose or hypromellose, or hydroxypropylcellulose and hypromellose.

5. The film preparation according to any one of claims 1 to 4, which is dissolved in an oral cavity.

6. The film preparation according to any one of claims 1 to 5, wherein the unpleasant taste derived from the medicament is masked.

7. A method for masking an unpleasant taste in a film preparation containing a medicament having an unpleasant taste, the method comprising forming coating layers containing no terpene on both sides of a medicament-containing layer containing a medicament having an unpleasant taste, and a terpene, wherein the unpleasant taste is a bitter taste, a harsh taste, or an astringent taste, wherein the content of the terpene in the film preparation is 0.2 to 15% by mass with respect to the whole film preparation, and wherein the medicament having an unpleasant taste is loxoprofen sodium hydrate or pitavastatin calcium.

8. A method for producing a film preparation in which an unpleasant taste, derived from a medicament is masked, the method comprising a step of forming coating layers containing no terpene on both sides of a medicament-containing layer containing a medicament having an unpleasant taste, and a terpene, wherein the unpleasant taste is a bitter taste, a harsh taste, or an astringent taste, wherein the content of the terpene in the film preparation is 0.2 to 15% by mass with respect to the whole film preparation, and wherein the medicament having an unpleasant taste is loxoprofen sodium hydrate or pitavastatin calcium.

## Patentansprüche

1. Filmpräparat, umfassend Überzugschichten, die kein Terpen enthalten, die auf beiden Seiten einer ein Medikament enthaltenden Schicht gebildet sind, die ein Medikament mit einem unangenehmen Geschmack und ein Terpen enthält, wobei der unangenehme Geschmack ein bitterer Geschmack, ein scharfer Geschmack oder ein adstringierender Geschmack ist, wobei der Gehalt des Terpens in dem Filmpräparat 0,2 bis 15 Massenprozent bezogen auf das gesamte Filmpräparat beträgt und wobei das Medikament mit dem unangenehmen Geschmack Loxoprofen-Natriumhydrat oder Pitavastatin-Calcium ist.

2. Filmpräparat nach Anspruch 1, wobei das Terpen Menthol ist.

3. Filmpräparat nach Anspruch 1 oder 2, wobei die das Medikament enthaltende Schicht oder die Überzugschichten oder die das Medikament enthaltende Schicht und die Überzugschichten ein schichtbildendes Mittel umfassen.

4. Filmpräparat nach Anspruch 3, wobei das schichtbildende Mittel Hydroxypropylcellulose oder Hypromellose oder Hydroxypropylcellulose und Hypromellose ist.

5. Filmpräparat nach einem der Ansprüche 1 bis 4, welches in der Mundhöhle gelöst wird.

6. Filmpräparat nach einem der Ansprüche 1 bis 5, wobei der unangenehme Geschmack, der von dem Medikament stammt, maskiert wird.

7. Verfahren zur Maskierung eines unangenehmen Geschmacks in einem Filmpräparat, das ein Medikament mit einem unangenehmen Geschmack enthält, wobei das Verfahren das Bilden von Überzugschichten, die kein Terpen enthalten, auf beiden Seiten einer ein Medikament enthaltenden Schicht, die ein Medikament mit einem unangenehmen Geschmack und ein Terpen enthält, umfasst, wobei der unangenehme Geschmack ein bitterer Geschmack, ein scharfer Geschmack oder ein adstringierender Geschmack ist, wobei der Gehalt des Terpens in dem Filmpräparat 0,2 bis 15 Massenprozent bezogen auf das gesamte Filmpräparat beträgt und wobei das Medikament mit dem unangenehmen Geschmack Loxoprofen-Natriumhydrat oder Pitavastatin-Calcium ist.

8. Verfahren zur Herstellung eines Filmpräparats, bei dem ein unangenehmer Geschmack, der von einem Medikament stammt, maskiert wird, wobei das Verfahren einen Schritt des Bildens von Überzugschichten, die kein Terpen enthalten, auf beiden Seiten einer ein Medikament enthaltenden Schicht, die ein Medikament mit einem unangenehmen Geschmack und ein Terpen enthält, umfasst, wobei der unangenehme Geschmack ein bitterer Geschmack, ein scharfer Geschmack oder ein adstringierender Geschmack ist, wobei der Gehalt des Terpens in dem Filmpräparat 0,2 bis 15 Massenprozent bezogen auf das gesamte Filmpräparat beträgt und wobei das Medikament mit dem unangenehmen Geschmack Loxoprofen-Natriumhydrat oder Pitavastatin-Calcium ist.

## Revendications

1. Préparation de film comprenant des couches de revêtement ne contenant pas de terpène formées sur les deux côtés d'une couche contenant un médicament qui contient un médicament ayant un goût déplaisant, et un terpène, dans laquelle le goût déplaisant est un goût amer, un goût âpre, ou un goût astringent, dans laquelle la teneur de la préparation de film en le terpène est de 0,2 à 15 % en masse par rapport à toute la préparation de film, et dans laquelle le médicament ayant un goût déplaisant est le loxoprofène sodique hydraté ou la pitavastatine calcique.

2. Préparation de film selon la revendication 1, dans laquelle le terpène est le menthol.

3. Préparation de film selon la revendication 1 ou 2, dans laquelle la couche contenant un médicament, ou les couches de revêtement, ou la couche contenant un médicament et les couches de revêtement, comprennent un agent filmogène.

4. Préparation de film selon la revendication 3, dans laquelle l'agent filmogène est l'hydroxypropylcellulose, ou l'hypromellose, ou l'hydroxypropylcellulose et l'hypromellose.

5. Préparation de film selon l'une quelconque des revendications 1 à 4, qui est dissoute dans la cavité orale.

6. Préparation de film selon l'une quelconque des revendications 1 à 5, dans laquelle le goût déplaisant dérivé du médicament est masqué.

7. Procédé pour masquer un goût déplaisant dans une préparation de film contenant un médicament ayant un goût déplaisant, le procédé comprenant la formation de couches de revêtement ne contenant pas de terpène sur les deux côtés d'une couche contenant un médicament qui contient un médicament ayant un goût déplaisant, et un terpène, dans lequel le goût déplaisant est un goût amer, un goût âpre, ou un goût astringent, dans lequel la teneur de la préparation de film en le terpène est de 0,2 à 15 % en masse par rapport à toute la préparation de film, et dans lequel le médicament ayant un goût déplaisant est le loxoprofène sodique hydraté ou la pitavastatine calcique.

8. Procédé pour produire une préparation de film dans laquelle un goût déplaisant, dérivé d'un médicament, est masqué, le procédé comprenant une étape de formation de couches de revêtement ne contenant pas de terpène sur les deux côtés d'une couche contenant un médicament qui contient un médicament ayant un goût déplaisant, et un terpène, dans lequel le goût déplaisant est un goût amer, un goût âpre, ou un goût astringent, dans lequel la teneur de la préparation de film en le terpène est de 0,2 à 15 % en masse par rapport à toute la préparation de film, et dans lequel le médicament ayant un goût déplaisant est le loxoprofène sodique hydraté ou la pitavastatine calcique.
